# NEUE EUROPÄISCHE PATENTSCHRIFT

(11) **EP 0 471 701 B2**
(45) Veröffentlichungstag und Bekanntmachung des Hinweises auf die Entscheidung über den Einspruch: **07.03.2007**
(45) Hinweis auf die Patenterteilung: 19.10.1994
(21) Anmeldenummer: 90906924.7
(22) Anmeldetag: 04.05.1990
(51) Int. Cl.: C07K 14/47, A61K 35/12

(54) **NEUE PROTEINE MIT TNF-HEMMENDER WIRKUNG UND IHRE HERSTELLUNG**
NOVEL TNF-INHIBIT PROTEINS AND THEIR PREPARATION
NOUVELLES PROTEINES A EFFET D'INHIBITION DE TNF ET LEUR PREPARATION

(30) Priorität: 09.05.1989 DE 3915072; 05.07.1989 DE 3922089
(43) Veröffentlichungstag der Anmeldung: 26.02.1992
(73) Patentinhaber: BASF Aktiengesellschaft, 67063 Ludwigshafen (DE)
(72) Erfinder: LEMAIRE, Hans-Georg, D-6716 Dirmstein (DE); HILLEN, Heinz, D-6733 Hassloch (DE); MOELLER, Achim, Winchester, MA 01890 (US); DAUM, Lothar, D-6701 Otterstadt (DE); DOERPER, Thomas, D-6719 Bissersheim (DE); SUBKOWSKI, Thomas, D-6704 Mutterstadt (DE)
(74) Vertreter: Schweiger, Georg
(86) Internationale Anmeldenummer: PCT/EP1990/000719
(87) Internationale Veröffentlichungsnummer: WO 1990/013575

(56) Entgegenhaltungen:
- EP-A- 0 308 378
- Chemical Abstracts, Band 108, No. 23, 6 June 1988, (Columbus, Ohio, USA), P Seckinger et al. Page 525, Zusammenfassung 203006c
- Chemical Abstracts, Band 110, No. 15, 10 April 1989 (Columbus, Ohio USA), C Peetre et al. Seite 553, Zusammenfassung 133375n
- Chemical Abstracts, Band 110, No. 23, 5 Juni 1989, (Columbus, Ohio, USA), I Olsson et al. Siehe Seite 557, Zusammenfassung 210604r
- TheJournal of Biological Chemistry, Band 264, No.20, 15 Juli 1989, American Society for Biochemistry and Molecular Biology, Inc., (US), P Seckinger et al.Seiten 11966-11973
- The Journal of Biological Chemistry, Band 264, No.20, 15 Juli 1989, American Society for Biochemistry and Molecular Biology, Inc., (US), H Engelmann et al. Seiten 11974-11980
- The Journal of Biological Chemistry, Band 265, No.3, 25 Januar 1990, American Society for Biochemistry and Molecular Biology, Inc., (US), H Engelmann et al. Seiten 1531-1536

## Beschreibung

Die vorliegende Erfindung betrifft neue Proteine und deren Herstellung.

TNFα (Tumor-Nekrose-Faktor) ist ein bekanntes Protein, welches ein breites Spektrum an biologischen Aktivitäten besitzt. Er beeinflußt verschiedene maligne und nicht-maligne Zelltypen, spielt eine Rolle bei septischem Schock und Gewebeverletzungen sowie Nierenabstoßungen, Transplantationen, Schocklunge und zerebraler Malaria (Lymphokines 1987 Vol. 14; Pharmaceutical Res. 5. 129 (1988); Science 234, 470 (1986); Nature 330, 662 (1987); J. Exp. Med. 166, 1132 (1987); Science 237, 1210 (1987); J. Exp. Med. 166, 1280 (1987)).

Es ist bekannt, daß man die Wirkung von TNFα mit Antikörpern neutralisieren kann (EP 260 610). Diese Antikörper sind jedoch nicht humane Substanzen, so daß sie bei der Anwendung am Menschen Immunreaktionen auslösen können.

Es wurden nun Proteine gefunden, die humanen Ursprungs sind und die Wirkung von TNFα neutralisieren können.

Gegenstand der Erfindung sind Proteine, welche ein Molekulargewicht von etwa 42.000 Dalton, bestimmt durch SDS-Gelelektrophorese, besitzen und deren Muteine, die durch geeignete Addition von Aminosäuren oder Peptiden oder durch Variation des Glykosidrestes entstehen, ohne daß dadurch die TNFα-inhibierende Wirkung der Proteine stark nachläßt;und wobei diese am N-Terminus die Aminosäuresequenz Xaa Thr Pro Tyr Ala Pro Glu Pro Gly Ser Thr Cys Arg Leu Arg Glu aufweisen, worin Xaa die Aminosäuresequenzen Gln Val Ala Phe, Ala Gln Val Ala Phe, Pro Ala Gln Val Ala Phe oder Leu Pro Ala Gln Val Ala Phe darstellt.

Die hier beschriebenen neuen Proteine besitzen saure Eigenschaften, ihr isoelektrischer Punkt liegt bei pH 2 bis 5. Sie binden sich spezifisch an TNFα und sind durch Trypsin schwer oder gar nicht verdaubar.

Die neuen Proteine lassen sich beispielsweise aus dem Harn von Patienten isolieren, die Fieber haben, d.h. deren Körpertemperatur etwa 38°C oder höher ist. Hierzu wird der Harn zunächst konzentriert, was beispielsweise durch Umkehrosmose oder Ultrafiltration geschehen kann. Das dadurch erhaltene Retentat wird anschließend durch lonenaustausch- und Affinitätschromatographie gereinigt.

Die Proteine lassen sich auch aus menschlicher Ascites-Flüssigkeit von Patienten mit Ovarialcarcinomen gewinnen.

Die Reinigung der Proteine kann nach bekannten Methoden, wie Affinitäts- oder Ionenaustauschchromatographie, erfolgen.

Die so gewonnenen Proteine sind am N-Terminus in der Aminosäuresequenz inhomogen. Solche Inhomogenitäten sind bei körpereigenen Proteinen nicht ungewöhnlich und treten beispielsweise auch beim γ-Interferon auf.

Durch Behandlung mit einer Endoglykosidase verändert das Protein sein Laufverhalten in der SDS-Polyacrylamidgel-Elektrophorese, was auf die Abspaltung von Zuckerresten zurückzuführen ist.

Die hier beschriebenen Proteine liegen im Urin und in der Ascitesflüssigkeit in Konzentrationen zwischen 1 - 100 µg/l vor. Um das Protein für pharmazeutische Zwecke in größeren Mengen verfügbar zu machen, kann man bekannte gentechnische Methoden (vgl. Maniatis, T. et al: Molecular Cloning: A Laboratory Manual, Cold Spring Harbor Press, N.Y., 1982) heranziehen. Für diesen Zweck muß zunächst die genetische Information für das neue Protein identifiziert und die entsprechende Nukleinsäure isoliert werden. Dazu wird das reine Protein mit Dithiothreitol reduziert, dann wird lodacetamid zur Derivatisierung der freien SH-Gruppen zugesetzt und anschließend wird das so behandelte Protein mit Bromcyan und anschließend mit Trypsin in kleine Peptide gespalten. Die Auftrennung der Peptide erfolgt über reversed phase-Chromatographie. Die N-terminale Sequenzierung eines dieser gereinigten Peptide ergab die Sequenz Val Phe Cys Thr Lys. Weiter enthält das Protein folgende drei weitere Peptidsequenzen: Gly Val Tyr Thr Ser, Ile Cys Thr Cys Arg Pro Gly Tyr und Pro Gly Thr Glu Thr Ser Asp Val Val Cys Lys Pro Cys Ala Pro Gly Thr Phe Ser Xab Thr Thr Ser Ser Asp Ile Cys Arg Pro, worin Xab eine noch unbekannte Aminosäure ist, die möglicherweise glykosyliert ist.

Die vorhandenen Peptidsequenzen erlauben nun durch die Synthese entsprechende Oligonukleotide eine eindeutige Identifizierung des Gens aus dem menschlichen Genom oder aus entsprechenden c-DNA-Bänken durch sequenzspezifische Filterhybridisierung.

Die so erhaltene genetische Information für das Protein kann dann in verschiedene Wirtszellen, wie eukaryontische Zellen, Hefen, Bacillus subtilis oder E. coli nach bekannten Methoden zur Expression gebracht und das Protein so erhalten werden. In den eukaryotischen Zellen entsteht dabei das Protein in glykosylierter Form.

Die Muteine, die sich durch Addition von Aminosäuren oder Peptiden von den neuen Proteinen ableiten, werden vorzugsweise nach gentechnischen Methoden dargestellt.

Die neuen Proteine zeigen gute TNFα-inhibierende Wirkungen und lassen sich daher zur Behandlung von Krankheiten einsetzen, bei denen die Konzentration an TNFα in Körperflüssigkeiten erhöht ist, wie z.B. bei septischem Schock. Weiter können sie bei folgenden Erkrankungen angewendet werden: Allergien, Autoimmunkrankheiten, Erkrankungen des rheumatischen Formenkreises, Schocklunge, entzündliche Knochenerkrankungen, Blutgerinnungsstörungen, Verbrennungen sowie bei Komplikationen nach Transplantationen.

### Beispiel 1

### Bestimmung der TNFα-inhibitorischen Wirkung

Die biologische Aktivität von TNFα wurde durch Lyse der Mauszellinie L929 (J. Biol. Chem. 260, 2345 (1985) und der Humanzellinie MCF7 bestimmt. Bei Versuchen zur Bestimmung der TNFα inhibitorischen Wirkung wurde eine Konzentration an TNFα gewählt, bei der mindestens 50 % der Zellen lysierten.

Überstände mit TNFα bindenden Proteinen wurden in 1:2 Schritten in Mikrotiterplatten verdünnt. Zu dieser Lösung (0,05 ml) wurden je 0.05 ml Human- bzw. Maus-TNFα (120 pg/ml) gegeben. Anschließend erfolgte die Zugabe von 50.000 L929-Zellen in 0,1 ml Medium, das 2 µg/ml Actinomycin D enthielt. Nach einer Inkubationszeit von 20-24 h bei 37°C im Brutschrank wurden die Zellen fixiert und mit Kristallviolett gefärbt. In Abwesenheit von TNFα-bindendem Protein lysierten TNFα und LT (Lymphotoxin) die Zellen. Diese wurden während der Färbung abgeschwemmt. Der schützende Effekt von überständen mit TNFα-bindenden Proteinen zeigte sich durch die Färbbarkeit von intakten Zellen, die zurückblieben.

Eine Inhibition der cytotoxischen Wirkung war sowohl gegenüber Human-TNFα, als auch etwas schwächer gegenüber Human-LT zu beobachten, nicht jedoch bei Maus-TNFα.

### Beispiel 2

### Proteinisolierung aus Urin

40 l Sammelurin von Patienten mit Fieber (≧38°C) wurde über eine Hemoflow^{®} F60 Patrone (Fa. Fresenius) filtriert, bis das Volumen des Retentatstromes auf 2,5 l aufkonzentriert war.

Das Retentat wurde anschließend zum Waschen 4 mal mit je 2,5 l 20 mM Natriumphosphatpuffer pH 4,0 versetzt und die Filtration jeweils bis zum Ausgangsvolumen von 2,5 l fortgesetzt.

Das so erhaltene proteinreiche, braun gefärbte Retentat wurde über S-Sepharose® der Fa. Pharmacia (Säule: Ø = 5 cm, l=17 cm) chromatographiert. Die Säule wurde vor Beginn des Auftrages mit 10 Säulenvolumina (SV) 20 mM Natriumphosphatpuffer, pH 5,5 (= Puffer I) äquilibriert und das Retentat aufgetragen. Es wurde mit 3 SV Puffer I nachgewaschen und das Wertprodukt durch Elution mit 3 SV eines 20 mM Natriumphosphatpuffers pH 6,5 (Puffer II) gewonnen.

Zur weiteren Reinigung wurde diese Fraktion über eine TNF-Affinitätssäule (Beispiel 4) gegeben (Ø = 1,5 cm, 1=10 cm), die mit 10 SV Puffer III (20 mM Natriumphosphat, 140 mM NaCl, pH 7,2) aquilibriert worden war. Nach dem Auftrag wurde mit 3 SV Puffer III nachgewaschen und die TNF-bindende Proteinfraktion durch Elution der Säule mit 40 ml eines Puffers IV, bestehend aus 0,58 % Essigsäure und 140 mM NaCl, gewaschen.

Zur Isolierung des reinen Proteins wurde das Eluat der TNF-Affinitätssäule über eine Mono Q-Säule HR 5/5 der Fa. Pharmacia aufgetrennt. Dazu wurde zunächst das Eluat mit 0,1 n NaOH auf pH 12,0 eingestellt.

Die Säule wurde mit 11 SV 20 mM Natriumphosphatpuffer pH 12,0 (Puffer V) äquilibriert. 10 ml des pH-adjustierten TNF-Affinitätssäuleneluates wurde aufgetragen und mit 4,4 SV Puffer V gewaschen. Anschließend wurde mit 20 mM Natriumphosphat pH 7,5, eluiert.

Zur weiteren Abreicherung von Verunreinigungen wurde die Mono Q-Säule mit 7 SV 20 mM Essigsäurepuffer gespült, der mit 0,1 N HCl auf pH 2,0 eingestellt worden war (Puffer VI).

Danach wurde die Säule mit 5-6 SV 20 mM Essigsäure, 20 mM NH₄Cl-Puffer, pH 2,0 (mit 0,1 n HCl eingestellt, Puffer VII) weiter eluiert. Nach 1-2 SV eluierte eine bei 280 nm UV-aktive Bande, die Verunreinigungen enthielt, nach weiteren 1-2 SV eluierte das neue Protein. Eine weitere Menge reines Protein kann durch Nachelution mit 1-2 SV eines auf 100 mM NaCl eingestellten Puffers VII erreicht werden.

Das Protein wurde so in einer gelelektrophoretischen Reinheit von >90 % erhalten. Aus 1 I Urin lassen sich etwa 1 bis 10 µg Protein erhalten.

### Beispiel 3

### Proteinisolierung aus menschlicher Ascitesflüssigkeit.

2,5 I leicht trübe, dünnflüssige Ascitesflüssigkeit, die als Punktat einer Patientin mit Ovarialcarcinom anfiel, wurde 30 min bei 3000 g zentrifugiert. Der überstand wurde mit 10 %iger Phosphorsäure auf pH 7,2 eingestellt und über eine mit Glutardialdehyd vernetzte TNF-Sepharose^{®}-Säule (vgl. Beispiel 4) gegeben. (Ø= 1,5 cm, I = 3 cm). Die Säule wurde mit 50 ml Puffer III äquilibriert und nach dem Auftrag mit 150 ml Puffer III nachgewaschen. Die TNF-bindenden Proteine wurden mit 30 ml Puffer IV eluiert.

Zur weiteren Reinigung wurde das Eluat mit 10%iger HCl auf pH 3,0 eingestellt und auf eine mit 20 mM Essigsäure (pH 3,0) äquilibrierte Chromatographiesäule (Mono S HR 5/5, Fa. Pharmacia) gegeben. Nach dem Auftrag wurde mit 10 ml 20 mM Essigsäure (pH 3,0) nachgewaschen und die TNF-bindenden Proteine anschließend durch Elution mit 4 ml eines Puffergemisches aus 6 Teilen 20 mM Essigsäure (pH 3,0) und 4 Teilen 50 mM Natriumphosphatpuffer (pH 9,0) eluiert. Der pH-Wert des Eluats wurde kontrolliert und gegebenenfalls auf pH 6,5 nachgestellt.

Das Eluat wurde Ober eine mit Natriumphosphatpuffer pH 6,0 (Puffer VIII) äquilibrierte Chomatographiesäule Mono Q HR 5/5 gegeben. Nach Waschen mit je 6 ml Puffer VIII und 6 ml 20 mM Essigsäure, 5 mM NaCl, pH 2,2 wurde das Protein mit 6 ml 20 mM Essigsäure, 150 mM NaCl, pH 2,0 (Puffer IX) von der Säule eluiert.

Die Charakterisierung des letzten Eluats ergab, daß es sich - abgesehen von der Inhomogenität der N-terminalen Sequenz - um das gleiche Protein handelt, welches gemäß Beispiel 2 erhalten wurde.

### Beispiel 4

### Herstellung der TNF-Affinitätssäule

a) Kopplung von TNF an BrCN-Sepharose
   7,5 g BrCN-Sepharose^{®} (Fa. Pharmacia) wurden in 30 ml Wasser aufgeschlemmt. Nach 30 min Quellzeit wurde die BrCN-Sepharose^{®}-Gelsuspension zuerst mit 500 ml 1 mM HCl-Lösung und dann mit 0,1 M NaHCO₃, 0,5 M NaCl, pH 8,3, gewaschen.
   136 mg TNF gelöst in 41 ml Puffer (0,1 M NaHCO₃, 0,5 M NaCl, pH 8.3) wurden zu dieser Gelsuspension gegeben. Der Reaktionsansatz wurde 2 h bei Raumtemperatur geschüttelt und die TNF-Sepharose^{®} bei 3000 U/min abzentrifugiert. Das Gelmaterial wurde mit 40 ml Puffer gewaschen.
   Aus der Proteinbestimmung der überstände errechnet sich eine Kopplungsausbeute von >90 %.
   Zur Blockierung der überschüssigen aktiven Gruppen der BrCN-Sepharose^{®} wurde die Gelsuspension mit 40 ml Puffer (0,1 M NaHCO₃, 0,5 M NaCl, 1 M Ethanolamin, pH 8,3) versetzt, anschließend 1 h bei Raumtemperatur geschüttelt und das Ethanolamin dann mit 3 x 40 ml Puffer (0,1 M NaHCO₃, 0,5 M NaCl, pH 8,3) ausgewaschen.
b) Vernetzung der TNF-Sepharose^{®} mit Glutardialdehyd
   20 ml nach a) hergestellter TNF-Sepharose^{®} Gelsuspension wurden zweimal mit 25 ml Puffer (20 mM Natriumphosphat, 140 mM NaCl, pH 8,0) gewaschen. Die Suspension wurde in 40 ml desselben Puffers aufgenommen und mit 1,6 ml 25 %iger Glutardialdehydlösung versetzt. Nach 1 h Schütteln bei Raumtemperatur wurde die Suspension abzentrifugiert und mit 25 ml Puffer (20 mM Natriumphosphat, 140 mM NaCl, 1 M Ethanolamin, pH 8,0) versetzt. Es wurde wieder 1 h geschüttelt und die TNF-Sepharose^{®} Suspension anschließend in eine Chromatographiesäule (Ø =1,5 cm, l =10 cm) gefüllt.
   Die Säule war nach Waschen mit 100 ml Puffer (20 mM Natriumphosphat, 140 mM NaCl, pH 7,2) und 50 ml 0,58 % Essigsäure + 140 mM NaCl für die Affinitätschromatographie einsetzbar.

### Beispiel 5

### Charakterisierung des Proteins

a) Molekulargewicht und Reinheit
   Zur Bestimmung des Molekulargewichts und der Reinheit wurden 2 µg des nach Beispiel 2 bzw. 3 erhaltenen Proteins einer 15 % SDS-Polyacrylamidgelelektrophorese unter reduzierenden und nicht reduzierenden Bedingungen unterworfen (Nature 227, 680 (1970)). Im Vergleich mit einer Reihe bekannter Eichproteine wurde das neue Protein nach beiden Methoden nach Färbung mit Coomassie blue als homogene Bande mit einem Molekulargewicht von etwa 42.000 Dalton erkannt.
   Andere Banden waren nicht zu erkennen. Die Reinheit des Proteins kann daher mit ≥90 % angegeben werden.
   Das Protein gibt sich als deutlich blau-violett gefärbte Bande zu erkennen.
b) N-terminale Sequenzierung
   10 µg (=250 pMol) des nach Beispiel 2 erhaltenen Proteins wurden mit einem Gasphasensequenzer N-terminal mehrmals sequenziert.
   Die N-terminale Sequenzanalyse deutet wegen des Auftretens verwandter Nebensequenzen auf die Inhomogenität der N-terminalen Aminosäuresequenz hin. Es wurden folgende Hauptsequenzen ermittelt:
   Sequenz 1a (nicht Gegenstand der Erfindung)
      Phe Thr Pro Tyr Ala Pro Glu Pro Gly Ser Thr Cys Arg Leu Arg Glu Nebenher konnte in der Gasphasensequenzierung eine um 6 Aminosäuren N-terminal verlängerte
   Sequenz 2a
      Leu Pro Ala Gln Val Ala Phe Thr Pro Tyr Ala Pro Glu Pro Gly Ser Thr Cys Arg Leu Arg Glu und eine um 1 Aminosäure N-terminal verminderte
   Sequenz 3a (nicht Gegenstand der Erfindung)
      Thr Pro Tyr Ala Pro Glu Pro Gly Ser Thr Cys Arg Leu Arg Glu
      ermittelt werden.
   Analog wurden in dem Protein gemäß Beispiel 3 folgender Hauptsequenzen ermittelt:
   Sequenz 1b
      Leu Pro Ala Gln Val Ala Phe Thr Pro Tyr Ala Pro Glu Pro Gly Ser Thr Cys Arg Leu Arg Glu
      (etwa 10 %)
   Sequenz 2b
      Pro Ala Gln Val Ala Phe Thr Pro Tyr Ala Pro Glu Pro Gly Ser Thr Cys Arg Leu Arg Glu
      (etwa 45 %)
   Sequenz 3b
      Ala Gln Val Ala Phe Thr Pro Tyr Ala Pro Glu Pro Gly Ser Thr Cys Arg Leu Arg Glu
      (etwa 45 %)
d) Behandlung mit Trypsin
   20 µg der neuen Proteine wurden bei pH 8,5 wie folgt behandelt:
   1. Zugabe von 0.5 µg Trypsin, gelöst in 0,1 M NaHCO₃-Puffer pH 8,5; Inkubation 16 h bei 37°C
   2. Zugabe von 0,5 µg Trypsin, gelöst in 0,1 % SDS - 0,1 M NaHCO₃-Puffer pH 8,5; Einstellen der Lösung auf 0,1 % SDS-Gehalt; Inkubation 16 h bei 37 °C.

   Die so behandelten Proteine wurden in einer 15 %igen SDS-Polyacrylamidelektrophorese vergleichend mit dem Ausgangsprotein analysiert. Es konnte kein Abbau der Proteine festgestellt werden.

### Beispiel 6

### Deglykosylierung

0,1 ml des gemäß Beispiel 2 erhaltenen Mono Q-Eluats ( 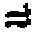 0,1 mg/ml Protein) wurden mit 1 M NaOH auf pH 7,2 eingestellt. Anschließend wurden 10 Units Glykopeptidase F (Fa. Boehringer Mannheim) zugesetzt. Nach 6 h Inkubation bei 37°C wurden weitere 10 Units Enzym zugesetzt. Nach weiteren 16 h Reaktionszeit wurden 50 µl des Ansatzes lyophilisiert und in einem 15%igen SDS-Gel im Vergleich mit unbehandelten Protein analysiert. Das mit Enzym behandelte Protein zeigte ein im Vergleich zur unbehandelten Probe um etwa 3 kD vermindertes Molekulargewicht. Weitere 25 µl des Ansatzes wurden, wie in Beispiel 1 beschrieben, auf TNFα inhibierende Wirkung getestet. Die TNFα-inhibierende Wirkung blieb auch nach Abspaltung des Zuckeranteils voll erhalten.

### Beispiel 7

### Antikörperproduktion

Die in den Beispielen 2 und 3 isolierten Proteine wurden zur Produktion polyklonaler Antikörper in Kaninchen injiziert. Die Reaktivität und Spezifität der Antikörper überprüfte man mittels ELISA. Dazu wurden ELISA-Platten (Fa. Costar) mit einer Lösung von 1 µg Inhibitor- bzw. Kontrollprotein/ml 0,05 M Natriumcarbonatpuffer pH 9,6 beschichtet, die unspezifische Bindung mit 1 % BSA/PBS abgesättigt und mit verschiedenen Serumverdünnungen inkubiert. Die Detektion der gebundenen Antikörper erfolgte mit biotinyliertem anti-rabbit-IgG und Streptavidin-Peroxidase, sowie TMB-Substrat. Zwischen den einzelnen Inkubationen wurde je 3mal mit 0,05 % ^{®}Tween-20/PBS gewaschen. Nach Abstoppen mit 2 M H₂SO₄ bestimmte man die optische Dichte bei 450 nm.

### Beispiel 8

### Proteinnachweis in Körperflüssigkeiten

Zum Nachweis von TNFα bindenden Proteinen in verschiedenen Körperflüssigkeiten diente ein sandwich-ELISA. Dazu wurden ELISA-Platten (Fa. Costar) mit TNF beschichtet (5 µg/ml 0,05 M Natriumcarbonatpuffer pH 9,6). Nach Absättigen mit 1 % BSA/PBS inkubierte man mit den zu untersuchenden Proben z.B. Synovialflüssigkeiten von Rheumatikern. Die Detektion erfolgte mit den unter Beispiel 7 beschriebenen anti-Inhibitor-Antikörpern und biotinyliertem anti-rabbit-lgG/Streptavidin-Peroxidase/TMB-Substrat. Zwischen den einzelnen Inkubationen wurde je 3mal mit 0.05 % ^{®}Tween-20/PBS gewaschen. Die Extinktion bei 450 nm wurde nach Zugabe von 2 M H₂SO₄ bestimmt.

## Patentansprüche

1. Proteine, welche ein Molekulargewicht von etwa 42.000 Dalton, bestimmt durch SDS-Gelelektrophorese, besitzen und deren Muteine, die durch geeignete Addition von Aminosäuen oder Peptiden oder durch Variation des Glykosidrestes entstehen, ohne daß **dadurch** die TNFα-inhibierende Wirkung der Proteine stark nachlässt; und wobei diese am N-Terminus die Aminosäuresequenz
Xaa Thr Pro Tyr Ala Pro Glu Pro Gly Ser Thr Cys Arg Leu Arg Glu
aufweisen, worin
Xaa
die Aminosäuresequenzen
Gln Val Ala Phe,
Ala Gln Val Ala Phe,
Pro Ala Gln Val Ala Phe oder
Leu Pro Ala Gln Val Ala Phe darstellt.

2. Proteine nach Anspruch 1 in deglykosylierter Form.

3. Verwendung der Proteine gemäß Anspruch 1 oder 2 zur Herstellung von Arzneimitteln zur Behandlung von Krankheiten, bei denen die Konzentration an TNF alpha in Körperflüssigkeiten erhöht ist.

4. Verwendung nach Anspruch 3, **dadurch gekennzeichnet, dass** die Krankheit eine Allergie, Autoimmunkrankheit, Erkrankung des rheumatischen Formenkreises, Schocklunge, entzündliche Knochenerkrankung, Blutgerinnungsstörung oder Komplikation nach Transplantationen ist.

5. Verwendung der Proteine gemäß Anspruch 1 oder 2 zur Herstellung von Antikörpern.

## Claims

1. Proteins which have a molecular weight of about 42,000 daltons, determined by SDS gel electrophoresis, and muteins thereof which are produced by suitable addition of amino acids or peptides or by altering the glycoside residue, without this leading to a large reduction in the TNF-inhibiting effect of the proteins, and where they have at the N terminus the amino acid sequence
Xaa Thr Pro Tyr Ala Pro Glu Pro Gly Ser Thr Cys Arg Leu Arg Glu
where Xaa is the amino acid sequences
Gln Val Ala Phe,
Ala Gln Val Ala Phe,
Pro Ala Gln Val Ala Phe or
Leu Pro Ala Gln Val Ala Phe.

2. Proteins according to claim 1 in deglycosylated form.

3. Use of the proteins according to Claim 1 or 2 for producing medicaments for the treatment of diseases in which the concentration of TNF alpha in body fluids is elevated.

4. Use according to Claim 3, **characterized in that** the disease is an allergy, autoimmune disease, rheumatic disorder, shock lung, inflammatory bone disorder, disturbance of blood clotting or complication following transplantations.

5. Use of the proteins according to Claim 1 or 2 for producing antibodies.

## Revendications

1. Protéine qui ont une masse molaire d'environ 42 000 daltons déterminée par électrophorèse surgel SDS, et leurs mutéines qui se forment par addition appropriée d'amino-acides ou de peptides ou par variation du reste glycoside sans que l'activité d'inhibition de TNF α des protéines ne baisse fortement celles-ci présentant sur le N terminal la séquence d'amino-acides
Xaa Thr Pro Tyr Ala Pro Glu Pro Gly Ser Thr Cys Arg Leu Arg Glu
dans laquelle Xaa présente les séquences d'aminoacides Gln Val Ala Phe, Ala Gln Val Ala Phe, Pro Ala Gln Val Ala Phe ou Leu Pro Ala Gln Val Ala Phe.

2. Protéines selon la revendication 1, sous forme déglycosylée.

3. Utilisation des protéines selon revendications 1 ou 2, pour la préparation de médicaments pour le traitement de maladies dans lesquelles la concentration en TNF α dans les fluides corporels est élevée.

4. Utilisation selon la revendication 3, **caractérisée en ce que** la maladie est une allergie, une maladie auto-immune, une maladie de type rhumatismal, une atteinte des poumons, une maladie osseuse inflammatoire, un trouble de la coagulation sanguine ou une complication après transplantation.

5. Utilisation des protéines selon les revendications 1 ou 2, pour la préparation d'anticorps.
